# EUROPEAN PATENT SPECIFICATION

(11) **EP 3 512 361 B1**
(45) Date of publication and mention of the grant of the patent: **10.04.2024**
(21) Application number: 17780630.4
(22) Date of filing: 13.09.2017
(51) Int. Cl.: A24F 40/42, A24D 3/06, A24D 3/04, A24F 40/10

(54) **A CONTAINER**
BEHÄLTER
CONTENANT

(30) Priority: 14.09.2016 GB 201615609
(43) Date of publication of application: 24.07.2019
(73) Proprietor: Nicoventures Trading Limited, London WC2R 3LA (GB)
(72) Inventor: HEPWORTH, Richard, London Greater London WC2R 3LA (GB)
(74) Representative: Harrison, Philip Mark
(86) International application number: PCT/EP2017/073061
(87) International publication number: WO 2018/050720

(56) References cited:
- EP-A1- 3 039 972
- EP-B1- 1 555 899
- WO-A1-2013/068100
- WO-A1-2014/158051
- WO-A2-2013/098405
- WO-A2-2016/135342
- US-A1- 2014 131 579
- US-A1- 2015 027 477

## Description

### Technical Field

The present invention relates to a container, and more particularly to a container for an apparatus for generating an inhalable material.

### Background

Smoking articles such as cigarettes, cigars and the like burn tobacco during use to create tobacco smoke. Attempts have been made to provide alternatives to these articles that burn tobacco by creating products that release compounds without burning. Examples of such products are heating devices which release compounds by heating, but not burning, the material. The material may be for example tobacco or other non-tobacco products, which may or may not contain nicotine.

As another example, there are so-called e-cigarette devices. These devices typically contain a liquid which is heated to vaporise the liquid to produce an inhalable vapour or aerosol. The liquid may contain nicotine and/or flavourings and/or aerosol-generating substances, such as glycerol. The known e-cigarette devices typically do not contain or use tobacco.
US 2015027477 A1 discloses a cigarette comprising a built-in capsule that incorporates a filter that incorporates a breakable capsule that contains a flavour enhancing liquid.

### Summary

According to a first aspect of the present invention, there is provided a substance container according to claim 1.

According to a second aspect of the invention, there is provided an aerosol provision device of claim 11.

According to a third aspect of the invention, there is provided the system of claim 15.

Further features and advantages of the invention will become apparent from the following description of preferred embodiments of the invention, given by way of example only, which is made with reference to the accompanying drawings. Like features appearing in different ones of the drawings are giving the same reference numerals in the different drawings.

### Brief Description of the Drawings

Figure 1 shows a schematic of an exemplary aerosol provision device;
Figure 2 shows a schematic sectional view of an example of a flavour container which is helpful for understanding of the invention;
Figure 3 shows a schematic sectional view of a flavour container according to the invention.

### Detailed Description

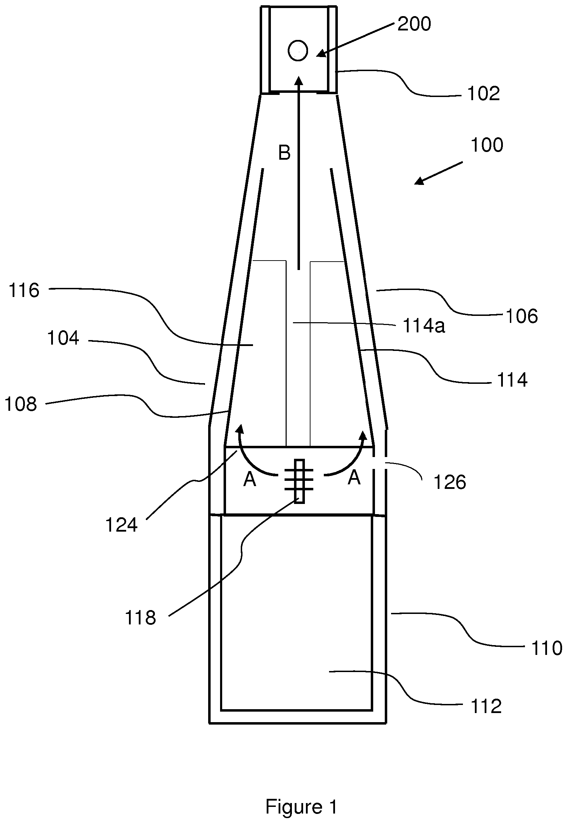

Referring to Figure 1, a schematic of an aerosol provision device 100 is illustrated. The aerosol provision device 100 is an inhalation device (i.e. a user uses it to inhale an aerosol provided by the device 100) and the device 100 is a hand held device. In this example, the device 100 is an electronic device, for example an electronic cigarette 100.

In broad outline, the device 100 volatilises a liquid to form a vapour or an aerosol which passes through a flavour container 200 received, for example, in a mouthpiece 102 of the device 100. In at least some examples a vapour is produced that then at least partly condenses to form an aerosol before exiting the device 100. The flavour container 200 imparts a flavour to the vapour or aerosol passing through the mouthpiece 102 for inhalation by a user.

In this respect, first it may be noted that, in general, a vapour is a substance in the gas phase at a temperature lower than its critical temperature, which means that for example the vapour can be condensed to a liquid by increasing its pressure without reducing the temperature. On the other hand, in general, an aerosol is a colloid of fine solid particles or liquid droplets, in air or another gas. A colloid is a substance in which microscopically dispersed insoluble particles are suspended throughout another substance.

For reasons of convenience, as used herein the term aerosol should be taken as meaning an aerosol, a vapour or a combination of an aerosol and vapour.

Returning to Figure 1, the device 100 comprises an outer body or housing 104 comprising an upper portion 106 housing a cartridge 108 and a lower portion 110 housing a battery portion 112. The cartridge 108 is connected to, but removable from, the battery portion 112. In this example, at least the upper portion 106 of the outer body 104 may be removed so as to expose the cartridge 108, and hence allow installation, removal and/or replacement of the cartridge 108. The cartridge 108 has a liquid container 114 for containing e-cigarette liquid 116.

The device 100 has the mouthpiece 102 removably connected to the upper portion 106 of the outer body 104. The mouthpiece 102 has received therein the flavour container 200 for imparting a flavour to a flow of aerosol that passes through the mouthpiece 102. Examples of flavour elements 200 will be discussed in more detail below.

The device 100 is arranged so that as the liquid 116 is volatilised so as to produce an aerosol at least some and preferably all or substantially all of the aerosol or passes through the flavour container 200 received in the mouthpiece 102 for example so as to entrain constituents of the flavour container 200 therein.

The liquid container 114 is provided generally centrally of the cartridge 108. The liquid container 114 is frusto-conical in shape, but may have a different shape, such as conical, cylindrical, etc. The liquid container 114 is annular in shape and defines a cylindrical channel 114a running through the length of the liquid container 114. The liquid container 114 may be formed of rigid, watertight and airtight materials, such as metal, suitable plastics, etc.

The cartridge 108 is provided with a heater 118 and a wick (not shown) in (thermal) contact with the heater 118. In this example, the heater 118 and the wick are provided as a single unit, sometimes known as an "atomiser". In this case, where the cartridge 108 includes an atomiser, such a cartridge 108 is often referred to as a "cartomiser".

The orientation of the heater 118 is shown schematically and for example the heater 118 may be a coil having its longitudinal axis perpendicular or parallel to the longitudinal axis of the cartridge 108.

The wick (not shown) is in contact with the liquid 116. This may be achieved, for example, by the wick (not shown) being inserted through a through hole (not shown) in an end wall 124 of the liquid container 114. Alternatively or additionally, the end wall 124 may be a porous member which allows liquid to pass through from the liquid container 114, and the wick (not shown) may be in contact with the porous end wall 124. The end wall 124 may be for example in the form of a porous ceramic disk. A porous end wall 124 of this type helps to regulate the flow of liquid onto the wick (not shown). The wick (not shown) is generally absorbent and acts to draw in liquid 116 from the liquid container 114 by capillary action (shown schematically in Fig. 1 by arrows A). The wick is preferably non-woven and may be for example a cotton or wool material or the like, or a synthetic material, including for example polyester, nylon, viscose, polypropylene or the like, or a ceramic material.

The cartridge 104 is (electrically) connected to a battery in the battery portion 112 to enable the heater 118 to be powered. When the heater 118 is powered (which may be instigated for example by the user operating a button (not shown) of the device 100 or by a puff detector (not shown) of the overall device 100, as is known per se), liquid 116 drawn (shown in Fig. 1 by arrows A) in from the liquid container 114 by the wick and is heated by the heater 118 to volatilise or vaporise the liquid, so as to generate an aerosol.

In use, the liquid 116 may be heated to a temperature of between around 100-300°C or more particularly around 150°C to 250°C. The liquid 116 may, or may not, comprise nicotine.

As the user draws on the mouthpiece 102, air is drawn through an air inlet 126. The liquid 116 is volatised or vaporised by the heater 110 into the air from the air inlet 126 thereby to produce a flow of an aerosol. The flow of aerosol is drawn through a channel 114a of the liquid container 114, through the flavour container 200 received in the mouthpiece 102, and out from the device 100 for inhalation by a user (this flow is shown by arrow B in Fig. 1).

The aerosol picks up (entrains) flavour (and/or other constituents) from a flavour substance in flavour container 200. One or more constituents of the flavour substance is thereby mixed with the flow of the aerosol thereby enhancing the sensory experience of a user. Advantageously, this enables a user to quickly add or modify a flavour sensation without having to first change the liquid 116 and in a way that reduces the likelihood of there being a thermal breakdown of that flavour that can sometimes occur with flavour provided in the actual liquid 116 following repeated use of the device 1.

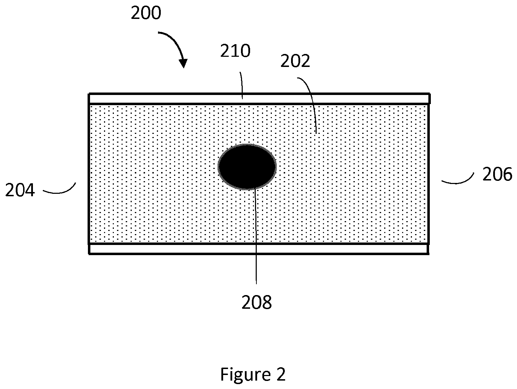

Figure 2 illustrates an example of a flavour container 200 that can be used in the device 100 as described above.

In this example, the flavour container 200 comprises an elongate body of material 202 the composition of which is such that, when in use in the device 100, the aerosol stream generated in the device 100 is able to enter the flavour container 200 at an 'upstream' end 204, pass through the length of the flavour container 200, and exit the flavour container 200 at a 'downstream' end 206. In other words, the elongate body of material 202 is permeable to the flow of aerosol generated in the device 100.

In some examples, the elongate body 202 comprises a fibrous material and may be, for example, a fibrous material that is typically used as a filter material in a traditional combustible cigarette, examples including cellulose acetate fibres, polypropylene fibres, polyster fibres and paper, including crimped paper. Other materials that may be used include, for example, nylon and the like.

In this example, a first flavour component 208 is supported in the elongate body of material 202.

In a preferred example, the first flavour component 208 is at least one flavour capsule that comprises an outer shell that encloses a flavour substance that comprises a flavourant. The flavour substance may be for example, a liquid, a gel, an emulsion, a suspension or beads. A user may, for example, manipulate the flavour container 200 in order to break the shell of the flavour capsule 208 in order to release the flavour substance into the material of the elongate body 202. In use, the flow of aerosol through the elongate body picks up (entrains) flavour (and/or other constituents) from the released flavour substance so that one or more constituents of the flavour substance is thereby mixed with the flow of the aerosol thereby enhancing the sensory experience of a user.

In an alternative example, the first flavour component 208 is again a flavour capsule that comprises an outer shell that encloses a substance that comprises a flavourant and, in use, the outer shell decomposes (e.g. melts) because of the heat of the aerosol flow to release the substance. Accordingly, in this example, there is no need for a user to manually break the capsule open.

In other examples, the flavour substance is not contained in a capsule. For example, the flavour substance may comprise a material that has been ground or otherwise treated so that it is in the form of particles, for example, powder, granules, grains, fibres, beads or the like particles which may be distributed substantially evenly throughout the elongate body of material 202 or concentrated in one or more particular sections of the body of material 202, for example the centre.

In some examples, such particles of flavour material may comprises tobacco although other botanicals or flavour agents may also be used.

In other examples, the flavour substance may comprises a gel or liquid or the like that is in body of material 202 but which is not released from a capsule.

In one example, the elongate body of material 202 comprises a continuous CA fibre formed into the elongate body of material 202 (e.g. the body of material 202 is what is known in the art as a 'dalmatian' type filter).

In another example, the elongate body of material 202 comprises a multiplicity of short cut CA fibres (e.g. fibres cut using a so called Turmalin apparatus) randomly orientated to form the elongate body of material 202.

An advantage of using short cut CA fibres is that a relatively small quantity of fibres are required to form the `rod like' shape of the elongate body of material 202 that supports the first flavour component 208. This provides the benefit of a low pressure drop of the aerosol flow across the elongate body of material 202. Additionally, less (or indeed no) plasticiser, for example, triacetine is required to maintain this rod form than is required when using a continuous fibre CA.

In some examples the length of the elongate body of material 202 is in the range 10 mm to 20 mm and preferably in the range 12 mm to 15 mm.

In some examples, the cross-sectional dimeter of the elongate body of material 202 is in the range 5 mm to 9 mm and preferably in the range 7.5 mm to 8 mm.

The tow cross section may, for example, be Y - shaped in cross section, X - shaped in cross section or Round shaped in cross section.

In some examples, when the flavour component 208 is a capsule, the capsule is substantially round 9 (i.e. spherical) in shape and its diameter is in the range 3 mm to 5 mm and preferably in the range of 3 mm to 4 mm. In other examples, when the flavour component 208 is a capsule, the capsule is substantially prolate (e.g. ellipsoid) in shape and its elongate length (i.e. that along its major axis) is in the range 3 mm to 5 mm and preferably in the range of 3 mm to 4 mm.

In some examples, the pressure difference across the length of the elongate body of material 202 when a user takes a draw (i.e. from the upstream end to the downstream end) is in the range of 0 to 40 mm water gauge (wg) or 0 to 30mm water gauge (wg), preferably in the range of 5 to 20 mm water gauge (wg) and most preferably is in the range 10 to 20mm wg. Alternatively, most preferably the pressure difference is in the range 5 to 15mm wg.

In some examples, when the elongate body of material 202 comprises CA, the CA fibre used has a single Denier value in the range of 8 - 12 for a single strand (i.e. the CA fibre has a mass in grams of between 8-12 per 9000 meters of the CA fiber). This range of Denier is particularly convenient for obtaining the above described pressure differences.

The flavour container 200 may further comprise an outer wrapper layer 210 that surrounds the elongate body of material 202 along its length to provide additional structural security to the flavour container 200. The outer wrapper layer 210 may comprise any suitable material, for example, paper. In some examples, the outer wrapper layer 210 is configured so that its acts as a liquid resistant barrier that prevents liquid, for example, condensation that forms around the flavour container 200 when it is in use, from getting into the interior of the flavour container 200 and making the flavour container soggy. In examples in which the flavour component 208 is a capsule that is manipulated by hand to release a flavour liquid or gel, the outer wrapper layer 210 prevents a user's fingers getting coated in the liquid or gel and acts to retain the liquid or gel in the flavour container 200. In one example, a liquid resistance wrapper layer 210 comprises paper impregnated with a barrier material or Natureflex or other suitable thin polymer film or plastic. The wrapper may have information printed on it, for example, an indication of a flavour type.

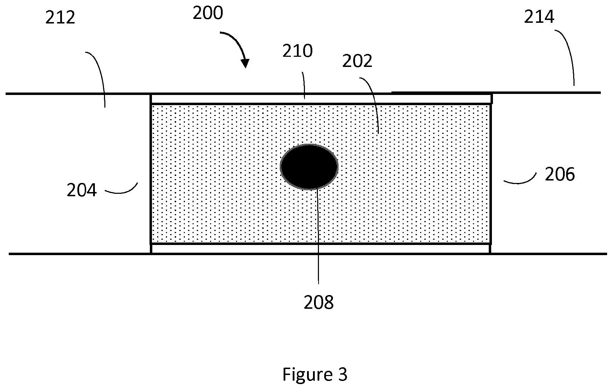

Referring now to Figure 3, the flavour container 200 comprises a hollow tube portion 212 extending from the region of the 'upstream' end 204 and a hollow tube portion 214 extending from the region of the 'downstream' end 206. The tube portion 212 extending from the region of the 'upstream' end 204 may be used to affix the flavour container 200 to the mouthpiece 102 of the smoking device 1. The hollow tube portion 214 extending from the region of the 'downstream' end 206 may itself be used as a mouthpiece by a user of the device 100. Either tube portion 212 or 214 may comprise any suitable material, for example, paper, cardboard, plastic or CA.

It is envisaged that after a certain number of draws, for example 20 - 50, the flavour of a flavour container 200 will be exhausted and at this point a user may replace a used flavour container 200 in a device 100 with a replacement flavour container 200. Replacement flavour containers 200 may of course be provided in packs. Each flavour container 200 may therefore be used over the course of multiple use sessions of the smoking device 1.

In some examples, sufficient liquid 116 is provided in the liquid container 114 to last multiple user sessions. In some examples, the liquid container 114 is arranged so that it can be re-filled or topped up with fresh liquid 116 by a user when the user so chooses. In other examples, a user cannot access the liquid container to 114 to add liquid to it and so in some examples, when the liquid 116 provided in the liquid container 114 is depleted or runs low a user may simply replace the used cartridge 108 with a new one.

In some examples, the cartridge 108 and the container 200 may be provided in combination together as a consumable that can be connected to, but removable from, the battery portion 112. This consumable may be designed to be a single session consumable (or at least limited number of sessions consumable) in that it contains sufficient liquid 116 and sufficient flavour substance in the container 200 to last a single user session (or limited number of user sessions) after which the user disposes of the consumable and replaces it with a new one.

Although in the examples described above, the container 200 is a flavour container and contains a substance for modifying a flavour of the aerosol when the aerosol flows through the body of material, this is not essential and instead (or in addition) the container 200 may contain a substance for modifying a property of the aerosol other than (or in addition) to flavour.

In some examples, the container 200 may contain a substance that modifies one or more other organoleptic properties of the aerosol (e.g. modifying the feel or smell or look of the aerosol to the user).

In some examples, the container 200 may contain a substance that modifies the PH of the aerosol by either lowering or raising the PH (e.g. modifying the acidity or the basicity of the aerosol).

In some examples, the container 200 may contain a substance that modifies (e.g. reduce) the amount of aldehydes in the aerosol.

In some examples, the container 200 may contain a substance that modifies different combinations of two or more of these or indeed other properties of the aerosol.

Although in the above described examples, the device 100 generates the aerosol by heating a liquid (the device is of type commonly referred to as an e-cig), this is not essential and in other examples, the device may generate the aerosol by heating, but not burning, a material, that may contain for example tobacco (e.g. a device sometimes referred to as a Tobacco Heating Product (THP) device).

Indeed, in any of the examples, the device 100 may include any suitable material for generating the aerosol and be in any suitable form, for example, a gel.

It will be appreciated that the container 200 may be provided in packs containing multiple containers

As used herein, the terms "flavour" and "flavourant" may refer to materials which, where local regulations permit, may be used to create a desired taste or aroma in a product for adult consumers. They may include extracts (e.g., licorice, hydrangea, Japanese white bark magnolia leaf, chamomile, fenugreek, clove, menthol, Japanese mint, aniseed, cinnamon, herb, wintergreen, cherry, berry, peach, apple, Drambuie, bourbon, scotch, whiskey, spearmint, peppermint, lavender, cardamom, celery, cascarilla, nutmeg, sandalwood, bergamot, geranium, honey essence, rose oil, vanilla, lemon oil, orange oil, cassia, caraway, cognac, jasmine, ylang-ylang, sage, fennel, piment, ginger, anise, coriander, coffee, or a mint oil from any species of the genus Mentha), flavour enhancers, bitterness receptor site blockers, sensorial receptor site activators or stimulators, sugars and/or sugar substitutes (e.g., sucralose, acesulfame potassium, aspartame, saccharine, cyclamates, lactose, sucrose, glucose, fructose, sorbitol, or mannitol), and other additives such as charcoal, chlorophyll, minerals, botanicals, or breath freshening agents. They may be imitation, synthetic or natural ingredients or blends thereof. They may be in any suitable form, for example, oil, liquid, solid, or powder. For example, a liquid, oil, or other such fluid flavourant may be impregnated in a porous solid material so as to impart flavour and/or other properties to that porous solid material. As such, the liquid or oil is a constituent of the material in which it is impregnated.

## Claims

1. A substance container (200) for an aerosol provision device (100) for providing an inhalable medium comprising an aerosol, the substance container (200) comprising:
a body of material (202) having a single fibre Denier value in the range of 8 to 12 that is permeable so as to allow the aerosol generated in the device (100) to flow into and through the body of material (202);
a substance in the body of material (202) for modifying a property of the aerosol when the aerosol flows through the body of material (202); a first tube portion (212) extending away from one end of the body of material (202) and a second tube portion (214) extending away from another end of the body of material (202).

2. The container (200) of claim 1 wherein the material is a fibrous material.

3. The container (200) of claim 1 or claim 2 wherein the material comprises at least one of CA, polypropylene, polyester and paper.

4. The container (200) of any of claims 1 to 3 wherein the length of the body of material (202) is in the range 10 mm to 20 mm and preferably in the range 12 mm to 15 mm.

5. The container (200) of any preceding claim wherein a cross section of the body of material (202) has a diameter that is in the range 5 mm to 9 mm and preferably in the range 7.5 mm to 8 mm.

6. The container (200) of any preceding claim further comprising a wrapping layer around the body of material (202) and/or the outer wrapping layer is moisture resistant.

7. The container (200) of any preceding claim further comprising at least one capsule comprising the substance.

8. The container (200) of claim 7 wherein the at least one capsule is breakable or squeezable to release the substance and/or the substance is a liquid or a gel.

9. The container (200) of any preceding claim wherein the substance is for modifying an organoleptic property of the aerosol and/or the substance is for modifying a flavour of the aerosol.

10. The container (200) of any preceding claim wherein the substance is for modifying the PH of the aerosol.

11. An aerosol provision device (100) for providing an inhalable medium comprising an aerosol; the device (100) comprising:
the container (200) of any of claims 1 to 10.

12. The device (100) according to claim 11, further comprising:
a container (200) for holding a liquid (116) or an aerosolisable material;
a heater for volatilising liquid (116) held in the container (200) to generate a flow of an aerosol in use or for heating but not combusting the aerosolisable material to generate a flow of an aerosol in use.

13. The device (100) of claim 11 or claim 12 further comprising:
a mouthpiece (102).

14. The device (100) of claim 13 wherein the container (200) is within or attached to the mouthpiece (102).

15. A system comprising the substance container (200) of any of claims 1 to 10 and a further container (200) for containing an aerosolisable material from which the aerosol is generatable.

## Patentansprüche

1. Substanzbehälter (200) für eine Aerosolbereitstellungsvorrichtung (100) zum Bereitstellen eines inhalierbaren Mediums, das ein Aerosol umfasst, wobei der Substanzbehälter (200) umfasst:
einen Materialkörper (202) mit einem Einzelfaser-Denier-Wert im Bereich von 8 bis 12, der durchlässig ist, um zu ermöglichen, dass das in der Vorrichtung (100) erzeugte Aerosol in und durch den Materialkörper (202) strömt;
eine Substanz im Materialkörper (202) zum Modifizieren einer Eigenschaft des Aerosols, wenn das Aerosol durch den Materialkörper (202) strömt; einen ersten Rohrabschnitt (212), der sich von einem Ende des Materialkörpers (202) weg erstreckt, und einen zweiten Rohrabschnitt (214), der sich von einem anderen Ende des Materialkörpers (202) weg erstreckt.

2. Behälter (200) nach Anspruch 1, wobei das Material ein faseriges Material ist.

3. Behälter (200) nach Anspruch 1 oder Anspruch 2, wobei das Material mindestens eines von CA, Polypropylen, Polyester und Papier umfasst.

4. Behälter (200) nach einem der Ansprüche 1 bis 3, wobei die Länge des Materialkörpers (202) der Bereich von 10 mm bis 20 mm ist und vorzugsweise im Bereich von 12 mm bis 15 mm liegt.

5. Behälter (200) nach einem der vorhergehenden Ansprüche, wobei ein Querschnitt des Materialkörpers (202) einen Durchmesser aufweist, der der Bereich von 5 mm bis 9 mm ist und vorzugsweise im Bereich von 7,5 mm bis 8 mm liegt.

6. Behälter (200) nach einem der vorhergehenden Ansprüche, ferner umfassend eine Umhüllungsschicht um den Materialkörper (202) und/oder die äußere Umhüllungsschicht ist feuchtigkeitsbeständig.

7. Behälter (200) nach einem der vorhergehenden Ansprüche, ferner umfassend mindestens eine Kapsel, die die Substanz umfasst.

8. Behälter (200) nach Anspruch 7, wobei die mindestens eine Kapsel zerbrechlich oder zusammendrückbar ist, um die Substanz freizusetzen, und/oder die Substanz eine Flüssigkeit oder ein Gel ist.

9. Behälter (200) nach einem der vorhergehenden Ansprüche, wobei die Substanz zum Modifizieren einer organoleptischen Eigenschaft des Aerosols vorgesehen ist und/oder die Substanz zum Modifizieren eines Aromas des Aerosols vorgesehen ist.

10. Behälter (200) nach einem der vorhergehenden Ansprüche, wobei die Substanz 1s zum Modifizieren des pH-Werts des Aerosols vorgesehen ist.

11. Aerosolbereitstellungsvorrichtung (100) zum Bereitstellen eines inhalierbaren Mediums, das ein Aerosol umfasst; wobei die Vorrichtung (100) umfasst:
den Behälter (200) nach einem der Ansprüche 1 bis 10.

12. Vorrichtung (100) nach Anspruch 11, ferner umfassend:
einen Behälter (200) zum Enthalten einer Flüssigkeit (116) oder eines aerosolisierbaren Materials;
eine Heizvorrichtung zum Verflüchtigen von Flüssigkeit (116), die in dem Behälter (200) enthalten ist, um im Gebrauch einen Strom eines Aerosols zu erzeugen, oder zum Erhitzen, aber nicht Verbrennen, des aerosolisierbaren Materials, um im Gebrauch einen Strom eines Aerosols zu erzeugen.

13. Vorrichtung (100) nach Anspruch 11 oder Anspruch 12, ferner umfassend:
ein Mundstück (102).

14. Vorrichtung (100) nach Anspruch 13, wobei sich der Behälter (200) im Mundstück (102) befindet oder daran befestigt ist.

15. System, umfassend den Substanzbehälter (200) nach einem der Ansprüche 1 bis 10 und einen weiteren Behälter (200) zum Enthalten eines aerosolisierbaren Materials, aus dem das Aerosol erzeugbar ist.

## Revendications

1. Contenant de substance (200) pour un dispositif de fourniture d'aérosol (100) destiné à fournir un milieu inhalable comprenant un aérosol, le contenant de substance (200) comprenant :
un corps de matériau (202) comportant une valeur Denier pour une seule fibre comprise dans la plage de 8 à 12 qui est perméable de manière à permettre à l'aérosol généré dans le dispositif (100) de s'écouler dans et à travers le corps de matériau (202) ;
une substance dans le corps de matériau (202) pour modifier une propriété de l'aérosol lorsque l'aérosol s'écoule à travers le corps de matériau (202) ; une première partie de tube (212) s'étendant en éloignement d'une extrémité du corps de matériau (202) et une seconde partie de tube (214) s'étendant en éloignement d'une autre extrémité du corps de matériau (202).

2. Contenant (200) selon la revendication 1, dans lequel le matériau est un matériau fibreux.

3. Contenant (200) selon la revendication 1 ou la revendication 2, dans lequel le matériau comprend au moins l'un parmi le CA, le polypropylène, le polyester et papier.

4. Contenant (200) selon l'une quelconque des revendications 1 à 3, dans lequel la longueur du corps de matériau (202) est compris la plage de 10 mm à 20 mm et de préférence dans la plage de 12 mm à 15 mm.

5. Contenant (200) selon l'une quelconque des revendications précédentes, dans lequel une section transversale du corps de matériau (202) comporte un diamètre compris la plage de 5 mm à 9 mm et de préférence dans la plage de 7,5 mm à 8 mm.

6. Contenant (200) selon l'une quelconque des revendications précédentes, comprenant en outre une couche d'enrobage autour du corps de matériau (202) et/ou la couche d'enrobage externe est résistante à l'humidité.

7. Contenant (200) selon l'une quelconque des revendications précédentes, comprenant en outre au moins une capsule comprenant la substance.

8. Contenant (200) selon la revendication 7, dans lequel l'au moins une capsule est cassable ou compressible pour libérer la substance et/ou la substance est un liquide ou un gel.

9. Contenant (200) selon une quelconque revendication précédente, dans lequel la première substance est destinée à modifier une propriété organoleptique de l'aérosol et/ou la substance est destinée à modifier un arôme de l'aérosol.

10. Contenant (200) selon l'une quelconque des revendications précédentes, dans lequel la substance est destinée à modifier le PH de l'aérosol.

11. Dispositif de fourniture d'aérosol (100) destiné à fournir un milieu inhalable comprenant un aérosol, le dispositif (100) comprenant :
le contenant (200) selon l'une quelconque des revendications 1 à 10.

12. Dispositif (100) selon la revendication 11, comprenant en outre :
un contenant (200) destiné à contenir un liquide (116) ou un matériau aérosolisable ;
un élément chauffant destiné à volatiliser le liquide (116) contenu dans le contenant (200) pour générer un écoulement d'un aérosol lors de l'utilisation ou pour chauffer mais sans brûler le matériau aérosolisable afin de générer un écoulement d'un aérosol lors de l'utilisation.

13. Dispositif (100) selon la revendication 11 ou la revendication 12, comprenant en outre : un embout buccal (102).

14. Dispositif (100) selon la revendication 13, dans lequel le contenant (200) est à l'intérieur ou fixé à l'embout buccal (102).

15. Système comprenant le contenant de substance (200) selon l'une quelconque des revendications 1 à 10 et un contenant supplémentaire (200) destiné à contenir un matériau aérosolisable à partir duquel l'aérosol peut être généré.
